Europäisches Patentamt

European Patent Office

Office européen des brevets

(11) Publication number: **0 313 939 B1**

## EUROPEAN PATENT SPECIFICATION

(45) Date of publication of patent specification: **16.09.92**

(51) Int. Cl.⁵: **C07C 309/04**, C07C 309/80

(21) Application number: **88117114.4**

(22) Date of filing: **14.10.88**

(54) Oxidation of thiols, disulfides and thiolsulfonates.

(30) Priority: **26.10.87 US 112648**

(43) Date of publication of application:
**03.05.89 Bulletin 89/18**

(45) Publication of the grant of the patent:
**16.09.92 Bulletin 92/38**

(84) Designated Contracting States:
**BE DE ES FR GB IT NL SE**

(56) References cited:
**FR-A- 2 299 320
US-A- 2 667 507
US-A- 3 626 004**

**R. BECKET et al.: "Methoden der Organischen Chemie", Houben-Weyl, vol. E11, 1st August 1985, page 1058, Georgh Thieme Verlag, Stuttgart, DE**

(73) Proprietor: **ATOCHEM NORTH AMERICA, INC. (a Pennsylvania corp.)
Three Parkway
Philadelphia Pennsylvania 19102(US)**

(72) Inventor: **Husain, Altaf
3313 E. Hayes Road
Norristown Pennsylvania 19401(US)**
Inventor: **Wheaton, Gregory Alan
130 Harvest Road
Swedesboro New Jersey 08085(US)**

(74) Representative: **Kraus, Walter, Dr. et al
Patentanwälte Kraus, Weisert & Partner
Thomas-Wimmer-Ring 15
W-8000 München 22(DE)**

Rank Xerox (UK) Business Services

## Description

This invention relates to the manufacture of alkanesulfonic acids and alkanesulfonyl chlorides by oxidation of the corresponding alkanethiol, dialkyldisulfide or alkyl alkanethiolsulfonate. More particularly, it relates to the oxidation of such corresponding compounds in mixtures of hydrogen peroxide and hydrogen chloride to form alkanesulfonic acids and alkanesulfonyl chloride free of undesirable side products arising from side-chain chlorination of the alkyl group as commonly observed in direct chlorine oxidation.

The most commonly used method for the manufacture of alkanesulfonic acids or alkanesulfonyl chlorides involves oxidation of corresponding alkanethiol or dialkyldisulfide by chlorine in concentrated hydrochloric acid media (e.g., U.S.3,626,004; U.S.4,280,966 and EPO,040,560) according to the following proposed equations:

$$RSH + 3Cl_2 + 2H_2O \rightarrow RSO_2Cl + 5HCl$$
$$RSH + 3Cl_2 + 3H_2O \rightarrow RSO_3H + 6HCl$$
$$RSSR + 5Cl_2 + 4H_2O \rightarrow 2RSO_2Cl + 8HCl$$
$$RSSR + 5Cl_2 + 6H_2O \rightarrow 2RSO_3H + 10HCl$$

A problem associated with the direct chlorine oxidation method is the formation of undesirable side-products arising from the chlorination of the alkyl side-chain. This problem becomes particularly serious in the manufacture of higher alkanesulfonic acids and alkanesulfonyl chlorides ($C_3$ and higher) due to the ease of direct side-chain chlorination. Because of the inherent thermal instability of alkanesulfonyl chlorides, it is extremely difficult to purify the crude product resulting from direct chlorine oxidation.

Another problem with the direct chlorine oxidation method is the large amount of by-product hydrochloric acid produced in the process. For each mole of alkanesulfonyl chloride, four and five moles of hydrochloric acid are produced using dialkyldisulfide and alkanethiol, respectively. Similarly, for each mole of alkanesulfonic acid, five and six moles of hydrochloric acid are produced using dialkyldisulfide and alkanethiol respectively. This causes a severe disposal problem both from economic and environmental considerations.

A method has previously been proposed to form ($C_4$-$C_{20}$) alkanesulfonyl chlorides with decreased side-products resulting from side-chain chlorination of the alkyl group wherein the corresponding alkanethiol or dialkyldisulfide is oxidized by a mixture of a small amount of oxygen in chlorine gas introduced to a mixture of a thiol or disulfide suspended in aqueous hydrogen chloride (See U.S.3,248,423) While this method apparently decreases the formation of unwanted side-products in the preparation of $C_4$ and higher alkanesulfonyl chlorides as compared to the direct chlorine oxidation method, appreciable quantities of the side-product are shown to be formed. In addition, this method suffers from the disadvantage that a large amount of hydrogen chloride is formed as a by-product of the oxidation reaction.

This invention is a process for preparing alkanesulfonic acids and alkanesulfonyl chlorides comprising contacting an alkanethiol, a dialkyldisulfide or an alkyl alkanethiolsulfonate in aqueous hydrochloric acid with hydrogen peroxide to produce the corresponding alkanesulfonic acid or alkanesulfonyl chloride.

The method of this invention involves oxidation of an organosulfur reactant selected from alkanethiols, dialkyldisulfides and alkyl alkanethiolsulfonates with a combination of hydrogen peroxide and hydrochloric acid according to the following chemical equations:

$$RSH + 3H_2O_2 + HCl \rightarrow RSO_2Cl + 4H_2O$$
$$RSSR + 5H_2O_2 + 2HCl \rightarrow 2RSO_2Cl + 6H_2O$$
$$RSO_2SR + 3H_2O_2 + 2HCl \rightarrow 2RSO_2Cl + 4H_2O$$
$$RSO_2Cl + H_2O \rightarrow RSO_3H + HCl$$

The alkanethiols that are employed in the present invention have 1-18 carbon atoms, preferably 1-8 carbon atoms. Thus, there is used, for example, methanethiol, ethanethiol, n-propanethiol, isopropanethiol, 1-butanethiol, 2-butanethiol, 1-hexanethiol, 1-octanethiol or 1-decanethiol. As the dialkyldisulfide, there is used, for example, compounds having 2-20 carbon atoms combined in the alkyl moieties, preferably 2-16 carbon atoms. For example, dimethyl disulfide, diethyl disulfide, dipropyl disulfide, diisopropyl disulfide, dibutyl disulfide, diamyl disulfide, dihexyl disulfide, dioctyldisulfide, or didecyl disulfide are used. As the alkyl alkanethiolsulfonate there is used, for example, compounds having 2-20 carbon atoms in the combined alkyl and alkane moieties, preferably 2-16 carbon atoms. For example, methyl methanethiolsulfonate, ethyl ethanethiolsulfonate, propyl propanethiolsulfonate, isopropyl isopropanethiolsulfonate, butyl butanethiolsulfonate, pentyl pentanethiolsulfonate, hexyl hexanethiolsulfonate, octyl octanethiolsulfonate and decyl decanethiolsulfonate are used.

The concentration of peroxide in the aqueous hydrogen peroxide solution that can be employed can range from 3 weight percent to 90 weight percent; however, concentrations of about 30 weight percent to 70 weight percent hydrogen peroxide, because of its availability, are preferred.

The amount of hydrogen peroxide used in the process of this invention can range from 3-4 moles for each mole of alkanethiol or alkyl alkanethiolsulfonate and 5-6 moles for each mole of dialkyldisulfide. Preferably, the amount of hydrogen peroxide used is about 3.3 moles for each mole of alkanethiol or alkyl alkanethiolsulfonate and about 5.5 moles for each mole of dialkyldisulfide.

The concentration of hydrogen chloride in the aqueous hydrochloric acid solution that can be employed is from 10 weight percent to 38 weight percent. Preferably, the concentration of hydrogen chloride is 36-38 weight percent.

The amount of hydrogen chloride used can range from 1-10 moles for each mole of alkanethiol or from 2-20 moles for each mole of dialkyldisulfide or alkyl alkanethiolsulfonate. Preferably, the amount of hydrogen chloride used is between about two and four moles for each mole of alkanethiol and between about four and six moles for each mole of dialkyldisulfide or alkyl alkanethiolsulfonate.

The temperature at which the process of this invention is carried out varies from 0°C to 60°C. Preferably the temperature is between 25-35°C for the preparation of alkanesulfonyl chloride and between 50-60°C for the preparation of alkanesulfonic acid.

The manner in which the process of this invention is carried out depends upon the individual organosulfur reactant employed and the product desired. In general, when alkanesulfonyl chloride is the desired product, aqueous hydrogen peroxide is added slowly to a mixture of alkanethiol (or dialkyldisulfide or alkyl alkanethiolsulfonate as the case may be) and aqueous hydrochloric acid over a period of one to two hours while the temperature is raised from 0°C to 35°C over the addition period. The reaction mixture is stirred at 35°C for an additional one or two hours. After this time, the reaction mixture is cooled and extracted with a suitable organic solvent such as, methylene chloride, chloroform, carbon tetrachloride, toluene, or equivalent solvent. The resultant organic extract is evaporated to obtain the alkanesulfonyl chloride. In most cases the product alkanesulfonyl chloride separates out as a lower layer. If desired the process of this invention can be run in a continuous manner by removing the lower product layer and charging fresh feed.

Similarly, if the desired product is alkanesulfonic acid, aqueous hydrogen peroxide is added to a mixture of alkanethiol (or dialkyldisulfide or alkyl alkanethiolsulfonate as the case may be) and aqueous hydrochloric acid over a period of one to two hours while the temperature is raised from 0°C to about 60°C over the addition period. The reaction mixture is stirred at 60°C for an additional one or two hours. The desired concentration of alkanesulfonic acid can then be produced from the product mixture by methods known to those skilled in the art.

The process of this invention is demonstrated in the following examples.

## Example 1

There was added, with vigourous stirring using a mechanical stirrer, 62.3g of 30 weight percent aqueous hydrogen peroxide (550 mmole) to a mixture of 9.4g of dimethyldisulfide (100 mmole) and 50g of 36.5 weight percent aqueous hydrochloric acid (500 mmole) over a period of one and one-half hours while the reaction temperature was increased from 5°C to 35°C. The reaction mixture was stirred at 35°C for an additional hour. The reaction mixture was then cooled to about 25°C and extracted with three 25ml aliquots of methylene chloride. Analysis of the methylene chloride extract by gas chromatography indicated that no detectable products arising from the side chain chlorination are formed. The organic extract was evaporated using a rotary evaporator, and the resulting product was distilled under reduced pressure to obtain 5.1g of pure methanesulfonyl chloride.

## Example 2

There was added, with vigorous stirring using a mechanical stirrer, 62.3g of 30 weight percent aqueous hydrogen peroxide (550 mmole) to a mixture of 15.0g of di-n-propyl-disulfide (100 mmole) and 50g of 36.5 weight percent aqueous hydrochloric acid (500 mmole) over a period of one and one-half hours while the reaction temperature was increased from 5°C to 35°C. The reaction mixture was stirred at 35°C for an additional one hour. The reaction mixture was then cooled and extracted with three 25ml portions of methylene chloride. Analysis of the methylene chloride extract by gas chromatography indicated that no detectable products arising from the side-chain chlorination are formed. From the methylene chloride extract, 16.9g (118.3 mmoles) of pure n-propanesulfonyl chloride was isolated in the same manner as in Example 1.

## Example 3

60g of 30 weight percent aqueous hydrogen peroxide (530 mmole) was added, with vigorous stirring using a mechanical stirrer, to a mixture of 23.4g of 1-octanethiol (160 mmole) and 50g of 36.5 weight percent aqueous hydrochloric acid (500 mmole) over a period of two hours while the reaction temperature was increased from 25°C to 50°C. The reaction mixture was stirred for an additional one hour at 50°C. The reaction mixture was then cooled and extracted with three 25ml portions of methylene chloride. Analysis of the methylene chloride extract by gas chromatography indicated that no detectable products arising from the side-

chain chlorination are formed. From the organic extract 14.7g of pure 1-octanesulfonyl chloride was obtained in the same manner as described in Example 1.

Example 4

There was added with vigorous stirring using a mechanical stirrer 62.3g of 30 weight percent aqueous hydrogen peroxide (550 mmoles) to a mixture of 9.4g of dimethyldisulfide (100 mmole) and 50g of 36.5 weight percent aqueous hydrochloric acid (500 mmole) over a period of one and one-half hours while the reaction temperature was increased from 5°C to 60°C. The reaction mixture was stirred at 60°C for an additional hour. After this time the reaction mixture was cooled to room temperature. In this manner 13.4g of methanesulfonic acid was obtained as analyzed by ion chromatography. No products arising from the side-chain chlorination are detected.

Example 5

There was added with vigourous stirring using a mechanical stirrer 37.4g of 30 weight percent aqueous hydrogen peroxide (330 mmoles) to a mixture of 12.6g of methyl methanethiolsulfonate (100 mmole) and 50g of 36.5 weight percent aqueous hydrochloric acid (500 mmoles) over a period of one and one-half hours while the reaction temperature was increased from 5°C to 35°C. The reaction mixture was stirred for an additional one hour at 35°C. After this stirring time, the reaction mixture was cooled and extracted with three 25ml. portions of methylene chloride. Analysis of the methylene chloride extract by gas chromatography indicated that no detectable products arising from side-chain chlorination are formed. From the organic extract, 15.63g of pure methanesulfonyl chloride was obtained in the same manner as described in Example 1.

For comparison with the results of the above examples, the following described reaction demonstrates the formation of side-chain chlorinated impurities in the preparation of n-propanesulfonyl chloride by reaction of n-propanethiol with chlorine in a concentrated aqueous hydrochloric acid medium as known in the prior art. n-propanethiol (5.50 gms) was added to 70 mls (80.90 gms) of concentrated hydrochloric acid (37.4 weight-% HCl) in a five-necked, tapered flask equipped with a sintered-glass gas dispersion tube, a mechanical stirrer, thermometer, and a reflux condenser. The flask was immersed in a water bath at 20°C and chlorine was introduced through the gas dispersion tube beneath the surface of the liquid at a rate of about 40 mls/min with vigorous mechanical mixing for a period of one hour. The product mixture was extracted with three 25 ml. portions of methylene chloride. Analysis of the organic extract by gas chromatography indicated that n-propanesulfonyl chloride was produced in 95% yield but that it contained 1.35 weight-% of a mixture of 1-chloropropanesulfonyl chloride and 3-chloropropanesulfonyl chloride as impurities.

## Claims

1. A process for preparing alkanesulfonic acids and alkanesulfonyl chlorides, characterized by contacting at a temperature from 0°C to 60°C an alkanethiol, a dialkyldisulfide or an alkyl alkanethiolsulfonate mixed in aqueous hydrochloric acid with hydrogen peroxide to produce the corresponding alkanesulfonic acid or alkanesulfonyl chloride.

2. The process of claim 1 wherein the alkanethiol has from 1 to 18 carbon atoms, the dialkyldisulfide has from 2 to 20 carbon atoms, and the alkyl alkanethiolsulfonate has from 2 to 20 carbon atoms.

3. The process of claim 1 wherein said hydrogen peroxide is used in an amount ranging from 3 to 4 moles for each mole of alkanethiol or alkyl alkanethiolsulfonate and from 5 to 6 moles for each mole of dialkyldisulfide.

4. The process of claim 3 wherein said hydrogen peroxide is the form of an aqueous solution at a concentration ranging from 3 to 90 percent based on the weight of the solution.

5. The process of claim 2 wherein the concentration of hydrogen chloride is from 10 to 38 percent based on the weight of the aqueous hydrochloric acid and the amount of hydrogen chloride used ranges from 1 to 10 moles for each mole of alkanethiol and from 2 to 20 moles for each mole of dialkyldisulfide or alkyl alkanethiolsulfonate.

6. The process of claim 5 wherein the hydrogen peroxide is in the form of an aqueous solution at a concentration ranging from 3 to 90 percent based on the weight of the solution and is present in an amount of from 3 to 4 moles for each mole of alkanethiol or alkyl alkanethiolsulfonate and from 5 to 6 moles for each mole of dialkyldisulfide.

7. The process of claim 6 wherein the alkanethiol has from 1 to 8 carbon atoms, the dialkyldisulfide has from 2 to 16 carbon atoms and the alkyl alkanethiolsulfonate has from 2 to 16 carbon atoms.

8. The process of claim 7 wherein the hydrogen peroxide concentration used in 30-70 weight percent and the amount of hydrogen peroxide present is about 3.3 moles, the hydrogen chloride concentration is 36 to 38 percent and the amount of hydrogen chloride used ranges from 2 to 4 moles for each mole of alkanethiol and 4 to 6 moles for each mole of dialkyldisulfide or alkyl alkanethiolsulfonate.

9. A process for preparing alkanesulfonic acids comprising contacting with hydrogen peroxide a mixture of an alkanethiol having from 1 to 8 carbon atoms, a dialkyldisulfide having from 2 to 16 carbon atoms or an alkyl alkanethiolsulfonate having from 2 to 16 carbon atoms with an aqueous hydrochloric acid solution having a hydrogen chloride concentration of 36 to 38 percent based on the weight of said solution and in an amount of 2 to 6 moles of hydrogen chloride per mole of alkanethiol, dialkyldisulfide or alkyl alkanethiolsulfonate, and the temperature of the process is between 50 and 60°C.

10. The process of claim 9 wherein the amount of hydrogen peroxide used is about 3.3 moles per mole of alkanethiol or alkyl alkanethiolsulfonate, or about 5.5 moles per mole of dialkyldisulfide.

11. The process of claim 10 wherein said hydrogen peroxide is in the form of an aqueous solution at a peroxide concentration of 30 to 70 percent based on the weight of said solution.

12. A process for preparing alkanesulfonyl chlorides comprising contacting with hydrogen peroxide a mixture of an alkanethiol having from 1 to 8 carbon atoms, a dialkyldisulfide having from 2 to 16 carbon atoms or an alkyl alkanethiolsulfonate having from 2 to 16 carbon atoms with an aqueous hydrochloric acid solution having a hydrogen chloride concentration of 36 to 38 percent based on the weight of said solution and in an amount of 2 to 6 moles of hydrogen chloride per mole of alkanethiol, dialkyldisulfide or alkyl alkanethiolsulfonate, and the temperatures of the process is between 25 and 35°C.

13. The process of claim 12 wherein the amount of hydrogen peroxide used is about 3.3 moles per mole of alkanethiol or alkyl alkanethiolsulfonate, or about 5.5 moles per mole of dialkyldisulfide.

14. The process of claim 13 wherein said hydrogen peroxide is in the form of an aqueous solution at a peroxide concentration of 30 to 70 percent based on the weight of said solution.

**Patentansprüche**

1. Verfahren zur Herstellung von Alkansulfonsäuren und Alkansulfonylchloriden, dadurch **gekennzeichnet,** daß bei einer Temperatur von 0°C bis 60°C ein Alkanthiol, ein Dialkyldisulfid oder ein Alkylalkanthiolsulfonat in wäßriger Salzsäure mit Wasserstoffperoxid unter Bildung der entsprechenden Alkansulfonsäure oder des Alkansulfonylchlorids behandelt werden.

2. Verfahren nach Anspruch 1, dadurch **gekennzeichnet,** daß das Alkanthiol 1 bis 18 Kohlenstoffatome besitzt, das Dialkyldisulfid 2 bis 20 Kohlenstoffatome besitzt, und das Alkylalkanthiolsulfonat 2 bis 20 Kohlenstoffatome besitzt.

3. Verfahren nach Anspruch 1, dadurch **gekennzeichnet,** daß das Wasserstoffperoxid in einer Menge im Bereich von 3 bis 4 mol für jedes mol Alkanthiol oder Alkylalkanthiolsulfonat und von 5 bis 6 mol für jedes mol Dialkyldisulfid verwendet wird.

4. Verfahren nach Anspruch 3, dadurch **gekennzeichnet,** daß das Wasserstoffperoxid in Form einer wäßrigen Lösung bei einer Konzentration im Bereich von 3 bis 90 %, bezogen auf das Gewicht der Lösung, verwendet wird.

5. Verfahren nach Anspruch 2, dadurch **gekennzeichnet,** daß die Konzentration an Chlorwasserstoff von 10 bis 38 %, bezogen auf das Gewicht der wäßrigen Salzsäure, beträgt, und die verwendete Menge an Chlorwasserstoff im Bereich von 1 bis 10 mol für jedes mol Alkanthiol und im Bereich von 2 bis 20 mol für jedes mol Dialkyldisulfid oder Alkylalkanthiolsulfonat, liegt.

6. Verfahren nach Anspruch 5, dadurch **gekennzeichnet,** daß das Wasserstoffperoxid in Form einer wäßrigen Lösung bei einer Konzentration im Bereich von 3 bis 90 %, bezogen auf das Gewicht der Lösung, vorliegt, und in einer Menge von 3 bis 4 mol für jedes mol Alkanthiol oder Alkylalkanthiolsulfonat und von 5 bis 6 mol für jedes mol Dialkyldisulfid, vorhanden ist.

7. Verfahren nach Anspruch 6, dadurch **gekennzeichnet,** daß das Alkanthiol 1 bis 8 Kohlenstoffatome enthält, das Dialkyldisulfid 2 bis 16 Kohlenstoffatome enthält und das Alkylalkanthiolsulfonat 2 bis 16 Kohlenstoffatome enthält.

8. Verfahren nach Anspruch 7, dadurch **gekennzeichnet,** daß die verwendete Wasserstoffperoxidkonzentration 30 bis 70 Gew.-% beträgt, und die vorliegende Menge an Wasserstoffperoxid etwa 3,3 mol beträgt, die Chlorwasserstoffkonzentration 36 bis 38 % beträgt, und die verwendete Menge an Chlorwasserstoff im Bereich von 2 bis 4 mol für jedes mol Alkanthiol und von 4 bis 6 mol für jedes mol Dialkyldisulfid oder Alkylalkanthiolsulfonat, liegt.

9. Verfahren zur Herstellung von Alkansulfonsäuren, dadurch **gekennzeichnet,** daß ein Gemisch aus einem Alkanthiol mit 1 bis 8 Kohlenstoffatomen, einem Dialkyldisulfid mit 2 bis 16 Kohlenstoffatomen oder einem Alkylalkanthiolsulfonat mit 2 bis 16 Kohlenstoffatomen mit einer wäßrigen Chlorwasserstofflösung mit einer Chlorwasserstoffkonzentration von 36 bis 38 %, bezogen auf das Gewicht der Lösung, und einer Menge von 2 bis 6 mol Chlorwasserstoff pro mol Alkanthiol, Dialkyldisulfid oder Alkylalkanthiolsulfonat mit Wasserstoffperoxid behandelt wird, wobei die Verfahrenstemperatur zwischen 50 und 60°C liegt.

10. Verfahren nach Anspruch 9, dadurch **gekennzeichnet,** daß die verwendete Menge an Wasserstoffperoxid etwa 3,3 mol pro mol Alkanthiol oder Alkylalkanthiolsulfonat, oder etwa 5,5 mol pro mol Dialkyldisulfid, beträgt.

11. Verfahren nach Anspruch 10, dadurch **gekennzeichnet,** daß das Wasserstoffperoxid in Form einer wäßrigen Lösung bei einer Peroxidkonzentration von 30 bis 70 %, bezogen auf das Gewicht der Lösung, verwendet wird.

12. Verfahren zur Herstellung von Alkansulfonylchloriden, dadurch **gekennzeichnet,** daß ein Gemisch aus einem Alkanthiol mit 1 bis 8 Kohlenstoffatomen, einem Dialkyldisulfid mit 2 bis 16 Kohlenstoffatomen oder einem Alkylalkanthiolsulfonat mit 2 bis 16 Kohlenstoffatomen mit einer wäßrigen Chlorwasserstofflösung mit einer Chlorwasserstoffkonzentration von 36 bis 38 %, bezogen auf das Gewicht der Lösung, und in einer Menge von 2 bis 6 mol Chlorwasserstoff pro mol Alkanthiol, Dialkyldisulfid oder Alkylalkanthiolsulfonat mit Wasserstoffperoxid behandelt wird, wobei die Verfahrenstemperatur zwischen 25 und 35°C liegt.

13. Verfahren nach Anspruch 12, dadurch **ge-**

**kennzeichnet,** daß die verwendete Menge an Wasserstoffperoxid etwa 3,3 mol pro mol Alkanthiol oder Alkylalkanthiolsulfonat, oder etwa 5,5 mol pro mol Dialkyldisulfid, beträgt.

14. Verfahren nach Anspruch 13, dadurch **gekennzeichnet,** daß das Wasserstoffperoxid in Form einer wäßrigen Lösung bei einer Peroxidkonzentration von 30 bis 70 %, bezogen auf das Gewicht der Lösung, vorliegt.

**Revendications**

1. Procédé de production d'acides alcanesulfoniques et de chlorures d'alcanesulfonyle, caractérisé par la mise en contact, à une température de 0 à 60°C, d'un alcanethiol, d'un disulfure de dialkyle ou d'un alcanethiolsulfonate d'alkyle en mélange dans de l'acide chlorhydrique aqueux avec du peroxyde d'hydrogène pour produire l'acide alcanesulfonique ou le chlorure d'alcanesulfonyle correspondant.

2. Procédé suivant la revendication 1, dans lequel l'alcanethiol a 1 à 18 atomes de carbone, le disulfure de dialkyle a 2 à 20 atomes de carbone et l'alcanethiolsulfonate d'alkyle a 2 à 20 atomes de carbone.

3. Procédé suivant la revendication 1, dans lequel le peroxyde d'hydrogène est utilisé en une quantité allant de 3 à 4 moles pour chaque mole d'alcanethiol ou d'alcanethiolsulfonate d'alkyle et de 5 à 6 moles pour chaque mole de disulfure de dialkyle.

4. Procédé suivant la revendication 3, dans lequel le peroxyde d'hydrogène est sous forme d'une solution aqueuse à une concentration allant de 3 à 90 % sur la base du poids de la solution.

5. Procédé suivant la revendication 2, dans lequel la concentration du chlorure d'hydrogène va de 10 à 38 % sur la base du poids de l'acide chlorhydrique aqueux et la quantité de chlorure d'hydrogène utilisée va de 1 à 10 moles pour chaque mole d'alcanethiol et de 2 à 20 moles pour chaque mole de disulfure de dialkyle ou d'alcanethiolsulfonate d'alkyle.

6. Procédé suivant la revendication 5, dans lequel le peroxyde d'hydrogène est sous forme d'une solution aqueuse à une concentration allant de 3 à 90 % sur la base du poids de la solution et est présent en une quantité de 3 à 4 moles pour chaque mole d'alcanethiol ou d'alcanethiolsulfonate d'alkyle et de 5 à 6 modes pour chaque mole de disulfure de dialkyle.

7. Procédé suivant la revendication 6, dans lequel l'alcanethiol a 1 à 8 atomes de carbone, le disulfure de dialkyle a 2 à 16 atomes de carbone et l'alcanethiolsulfonate d'alkyle a 2 à 16 atomes de carbone.

8. Procédé suivant la revendication 7, dans lequel la concentration du peroxyde d'hydrogène utilisée va de 30 à 70 % en poids et la quantité de peroxyde d'hydrogène présente est d'environ 3,3 moles, la concentration du chlorure d'hydrogène est de 36 à 38 % et la quantité de chlorure d'hydrogène utilisée va de 2 à 4 moles pour chaque mole d'alcanethiol et de 4 à 6 moles pour chaque mole de disulfure de dialkyle ou d'alcanethiolsulfonate d'alkyle.

9. Procédé de production d'acides alcanesulfoniques, qui consiste à faire entrer en contact avec du peroxyde d'hydrogène un mélange d'un alcanethiol ayant 1 à 8 atomes de carbone, d'un disulfure de dialkyle ayant 2 à 16 atomes de carbone ou d'un alcanethiolsulfonate d'alkyle ayant 2 à 16 atomes de carbone et d'une solution aqueuse d'acide chlorhydrique ayant une concentration en chlorure d'hydrogène de 36 à 38 % sur la base du poids de ladite solution et en une quantité de 2 à 6 moles de chlorure d'hydrogène par mole d'alcanethiol, de disulfure de dialkyle ou d'alcanethiolsulfonate d'alkyle, et la température du procédé se situe entre 50 et 60 °C.

10. Procédé suivant la revendication 9, dans lequel la quantité de peroxyde d'hydrogène utilisée est d'environ 3,3 moles par mole d'alcanethiol ou d'alcanethiolsulfonate d'alkyle ou d'environ 5,5 moles par mole de disulfure de dialkyle.

11. Procédé suivant la revendication 10, dans lequel le peroxyde d'hydrogène est sous la forme d'une solution aqueuse ayant une concentration en peroxyde d'hydrogène de 30 à 70 % sur la base du poids de ladite solution.

12. Procédé de production de chlorures d'alcanesulfonyle, qui consiste à faire entrer en contact avec du peroxyde d'hydrogène un mélange d'un alcanethiol ayant 1 à 8 atomes de carbone, d'un disulfure de dialkyle ayant 2 à 16 atomes de carbone ou d'un alcanethiolsulfonate d'alkyle ayant 2 à 16 atomes de carbone et d'une solution aqueuse d'acide chlorhydrique ayant une concentration en chlorure d'hydrogène de 36 à 38 % sur la base du poids de ladite solution et en une quantité de 2 à 6 moles de chlorure d'hydrogène par mole d'alcanethiol, de disulfure de dialkyle ou d'alcanethiolsulfonate d'alkyle, et la température du procédé se situe entre 25 et 35 °C.

13. Procédé suivant la revendication 12, dans lequel la quantité de peroxyde d'hydrogène utilisée est d'environ 3,3 moles par mole d'alcanethiol ou d'alcanethiolsulfonate d'alkyle, ou d'environ 5,5 moles par mole de disulfure de dialkyle.

14. Procédé suivant la revendication 13, dans lequel le peroxyde d'hydrogène est sous forme d'une solution aqueuse ayant une concentration en peroxyde de 30 à 70 % sur la base du poids de ladite solution.